# EUROPEAN PATENT SPECIFICATION

(11) **EP 2 515 865 B1**
(45) Date of publication and mention of the grant of the patent: **14.12.2016**
(21) Application number: 10803218.6
(22) Date of filing: 22.12.2010
(51) Int. Cl.: A61K 9/06, A61K 31/593, A61K 47/10, A61K 47/14, A61K 47/22, A61K 9/107

(54) **CUTANEOUS COMPOSITION COMPRISING VITAMIN D ANALOGUE AND A MIXTURE OF SOLVENT AND SURFACTANTS**
ZUSAMMENSETZUNG FÜR DIE HAUT MIT VITAMIN-D-ANALOGON UND EINER MISCHUNG AUS EINEM LÖSUNGSMITTEL UND TENSIDEN
COMPOSITION CUTANÉE COMPRENANT UN ANALOGUE DE LA VITAMINE D ET UN MÉLANGE DE SOLVANT ET DE TENSIOACTIFS

(30) Priority: 22.12.2009 WO PCT/DK2009/000265; 07.01.2010 US 293105 P
(43) Date of publication of application: 31.10.2012
(73) Proprietor: Leo Pharma A/S, 2750 Ballerup (DK)
(72) Inventor: PETERSSON, Karsten, DK-2750 Ballerup (DK)
(74) Representative: Thalsoe-Madsen, Kine Birgit
(86) International application number: PCT/DK2010/000182
(87) International publication number: WO 2011/076207

(56) References cited:
- WO-A1-2006/134272
- WO-A2-2008/065316
- US-A1- 2006 189 586
- ANONYMOUS: 'Paruvirku Ennai' TKDL 01 January 2002, XP003029304
- ANONYMOUS: 'Snuhyadyamtailam (01)' TKDL 01 August 1999, XP003029241
- ANONYMOUS: 'Snukksarayogah' TKDL 01 January 1999, XP003029303
- ANONYMOUS: 'Mukhdusikahara Lepa' TKDL 01 August 1998, XP003029239
- ANONYMOUS: 'Tila Reebaas Bara-e- Jarb-o- Hikka' TKDL 01 January 1968, XP003029305
- ANONYMOUS: 'Roughan-e-arand Barae Tila' TKDL 01 January 1926, XP003029302
- ANONYMOUS: 'Dawa-e-roghan-e-khopra Brae Jild' TKDL 01 January 1926, XP003029234
- ANONYMOUS: 'Zaitoon' TKDL 01 January 1874, XP003024312
- BÄUMER, WOLFGANG; HOPPMANN, JOACHIM; RUNDFELDT, CHRIS; KIETZMANN, MANFRED: "Highly selective phosphodiesterase 4 inhibitors for the treatment of allergic skin diseases and psoriasis", INFLAMMATION AND ALLERGY - DRUG TARGETS, vol. 6, 2006, pages 17-26,

## Description

### FIELD OF INVENTION

The present invention relates to a cutaneous pharmaceutical composition which comprises a vitamin D analogue as a therapeutically active compound and a mixture of a solvent and surfactants in a pharmaceutically acceptable carrier.

### BACKGROUND OF THE INVENTION

Psoriasis is a chronic inflammatory skin disease that manifests as erythematous, dry, scaling plaques resulting from hyperkeratosis. The plaques are most often found on the elbows, knees and scalp, though more extensive lesions may appear on other parts of the body, notably the lumbosacral region. The most common treatment of mild to moderate psoriasis involves topical application of a composition containing a corticosteroid as the active ingredient. While efficacious, corticosteroids have the disadvantage of a number of adverse effects such as skin atrophy, striae, acneiform eruptions, perioral dermatitis, overgrowth of skin fungus and bacteria, hypopigmentation of pigmented skin and rosacea.

For many years, however, an advantageous non-steroidal treatment of psoriasis has consisted in topical treatment with the vitamin D analogue compound, calcipotriol, formulated in an ointment composition (marketed as Daivonex^{®} or Dovonex^{®} ointment by LEO Pharma) in which the calcipotriol is present in solution or a cream composition (marketed as Daivonex^{®} or Dovonex^{®} cream by LEO Pharma) in which the calcipotriol is present as a suspension. The solvent in the ointment composition is propylene glycol which has the advantage of enhancing penetration of the active ingredient into the skin, leading to an improved efficacy, but which is also known to act as a skin irritant. Thus, it has been reported that the inclusion of propylene glycol in topical compositions frequently causes patients to develop contact dermatitis (one study reported a number of irritant reactions to propylene glycol of 12.5%, cf. M. Hannuksela et al., Contact Dermatitis 1, 1975, pp. 112-116), and the number of irritant reactions increases when propylene glycol is used in high concentrations (as reviewed by J. Catanzaro and J. Graham Smith, J. Am. Acad. Dermatol. 24, 1991, pp. 90-95). Due to the improved penetration of calcipotriol into the skin resulting, *inter alia,* from the presence of propylene glycol, Daivonex^{®} ointment has been found to be more efficacious in the treatment of psoriatic lesions than Daivonex^{®} cream, but has also caused skin irritation in a significant proportion of psoriasis patients. WO 2008/065316 A2 discloses further topical compositions comprising a vitamin D derivative.

It is therefore an object of the invention to provide a topical composition comprising a vitamin D derivative or analogue as the active ingredient, which has skin penetration and biological activity properties comparable to those of Daivonex^{®} ointment, but which does not contain propylene glycol as the solvent.

### SUMMARY OF THE INVENTION

Human skin, in particular the outer layer, the stratum corneum, provides an effective barrier against penetration of microbial pathogens and toxic chemicals. While this property of skin is generally beneficial, it complicates the dermal administration of pharmaceuticals in that a large quantity, if not most, of the active ingredient applied on the skin of a patient suffering from a dermal disease may not penetrate into the viable layers of the skin where it exerts its activity. To ensure adequate penetration of the active ingredient to the dermis and epidermis, it is generally preferred to include the active ingredient in a dissolved state, typically in the presence of a solvent in the form of an alcohol, e.g. ethanol, or diol, e.g. propylene glycol. Propylene glycol is a well-known penetration enhancer, i.e. a substance which is capable of penetrating the stratum corneum and "draw" low-molecular components such as therapeutically active components in the vehicle into the epidermis. Propylene glycol may in itself give rise to significant skin irritation, and it is also capable of "drawing" low-molecular and potentially irritative components of the vehicle into the epidermis, leading to an overall irritative effect of conventional vehicles including propylene glycol. For this reason, the presence of propylene glycol as a solvent in compositions intended for the treatment of inflammatory skin diseases may exacerbate the inflammatory response.

In the research leading to the present invention, it was surprisingly found that certain three-component surfactant-solvent mixtures of a type which self-emulsifies in the presence of an excess of water to form microemulsions are suitable for inclusion in topical compositions for application on skin. The mixtures also exhibit a good solubilization capacity in dissolving sparingly water-soluble active ingredients such as vitamin D derivatives and analogues. The compositions are easily spreadable, and therefore likely to improve patient compliance, and exhibit an adequate physical and chemical stability. Compositions according to the invention comprising a vitamin D derivative or analogue have surprisingly been found to lead to a very high activation of the target gene cathelicidin in the biological assay described in Example 7 below, suggesting that the active ingredient is internalized by the keratinocytes on which the compositions are applied and activates the vitamin D receptor to a higher than usual degree. Without wishing to be limited to any particular theory, it is currently assumed that the three-component surfactant-solvent mixture, when it permeates the viable skin layers, modifies the cell wall of the keratinocytes in such a way that the vitamin D derivative or analogue is more readily taken up by the cells. While it might be expected that the higher biological activity presumed to be the result of cell wall modification would lead to increased skin irritation, this was not apparent when a composition of the invention was tested in a local tolerance study in minipigs, cf. Example 8, or in human volunteers.

Accordingly, the present invention relates to a substantially anhydrous pharmaceutical composition for cutaneous application comprising 1-20% by weight of an isotropic solvent mixture of
(a) a hydrophilic non-ionic surfactant which is a polyethylene glycol ester of a vegetable oil containing at least 20 mole of ethylene oxide groups/mole of glyceride;
(b) a lipophilic non-ionic co-surfactant which is selected from the group consisting of monoglyceride esters of C6 -22 fatty acids, diglyceride esters of C6 -22 fatty acids, mono-and diglyceride esters of C6 -22 fatty acids, propylene glycol esters of C6 -22 fatty acids, dialkylene glycol monoalkyl ethers, or polyethylene glycol esters of a triglyceride/vegetable oil containing 4-8 mole of ethylene oxide groups/mole of glyceride;
(c) a C6 -22 acylglyceride which is a non-amphiphilic C6 -22 acylglyceride selected from the group consisting of highly purified vegetable oils such as medium chain triglycerides, long chain triglycerides or castor oil;
said isotropic solvent mixture being capable of forming a microemulsion in the presence of an excess of water;
the composition further comprising a vitamin D derivative or analogue dissolved or solubilized in said isotropic solvent mixture, the vitamin D derivative or analogue being selected from the group consisting of calcipotriol, calcitriol, tacalcitol, maxacalcitol, paricalcitol and alfacalcidol,
and a pharmaceutically acceptable, substantially anhydrous lipid carrier which is a paraffin selected from hydrocarbons with chain lengths from C5 to C60.

Solvent mixtures of the type included in the present compositions have been described in the literature. Thus, US 5,645,856 discloses a pharmaceutical composition comprising a hydrophobic drug, a digestible oil, a hydrophilic surfactant and a lipophilic surfactant. The composition is intended to increase the solubility of the hydrophobic drug on oral administration in that the oil-surfactant mixture self-emulsifies in gastric fluid resulting in the formation of a microemulsion claimed to result in faster and more complete absorption of the drug. There is no indication that that the solvent mixtures disclosed in US 5,645,856 could be incorporated in compositions intended for dermal application. US 5,948,825 discloses a water-in-oil microemulsion comprising an oil phase, an aqueous phase and a combination of hydrophilic and lipophilic surfactants, the dispersed oil droplets of the microemulsion having a particle size of 0.4-100 nm. Said microemulsions are intended for systemic delivery of pharmaceutically active proteins dissolved in an aqueous phase, or to improve the bioavailability of low molecular weight drugs. There is no indication that the microemulsions disclosed in US 5,948,825 could be incorporated in a composition intended for dermal application.

US 6,267,985 discloses a composition comprising a triglyceride and either two hydrophilic surfactants or one hydrophilic and one lipophilic surfactant as well as an active ingredient solubilized in the triglyceride or triglyceride-surfactant mixture. The composition forms a clear aqueous dispersion when mixed with water in a ratio of 1:100. The composition is intended for oral administration to provide improved absorption of the active ingredient in the gastrointestinal tract. There is no suggestion of mixing the composition with excipients that would make it appropriate for cutaneous application.

M. Grove et al., European Journal of Pharmaceutical Sciences 28, 2006, pp. 233-242, disclose a drug delivery system comprising a lipid, surfactant and co-surfactant as well as a vitamin D analogue (seocalcitol) as the active ingredient. On dilution with water, the system formed microemulsions with a droplet size of 30 nm. On oral administration to rats, the bioavailability of seocalcitol was not improved over a formulation in lipid alone, and the chemical stability had decreased below the acceptable limit after 3 months at 40°C/75% RH. There is no indication that the drug delivery system disclosed in Grove et al. is suitable of incorporation in a composition intended for dermal application or that it may be possible to obtain an adequate chemical stability of the vitamin D analogue included in such a composition.

The composition of the invention differs from those disclosed in these publications by being intended for cutaneous application and by comprising one or more excipients that are suitable for dermal use. In particular, the substantially anhydrous lipid carrier is expected to provide an occlusive layer on the skin surface on which the composition is applied such that moisture evaporating or secreted from the skin accumulates between the skin surface and the occlusive layer. While the amount of moisture is not expected to be sufficient to cause self-emulsification of the isotropic solvent mixture to form a microemulsion, such as is disclosed in the publications mentioned above, it is presumed to result in the formation of ordered structures such as liquid crystalline, lamellar phases or micelles comprising the solubilized or dissolved active ingredient, depending on the amount of water present. The presence of surfactant and co-surfactant in the composition may contribute to the penetration of the active ingredient as the surfactant(s) may modulate the cellular membrane to increase its permeability to small chemical entities such as vitamin D derivatives or analogues.

In another aspect, the invention relates to a pharmaceutical composition as described herein for use in the prevention or treatment of dermal diseases or conditions.

### BRIEF DESCRIPTION OF THE DRAWINGS

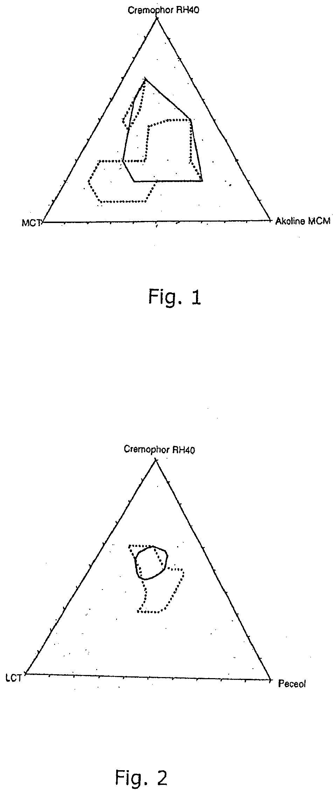

Fig. 1 is a ternary phase diagram for an isotropic solvent mixture comprising MCT with Cremophor RH40 and Akoline MCM. The solid line represents the area where a microemulsion is formed on addition of 250 ml of water to 1 gram of the mixture, and the dotted line represents the areas where the mixture is monophasic.
Fig. 2 is a ternary phase diagram for an isotropic solvent mixture comprising LCT with Cremophor RH40 and Peceol. The solid line represents the area where a microemulsion is formed on addition of 250 ml of water to 1 gram of the mixture, and the dotted line represents the areas where the mixture is monophasic.

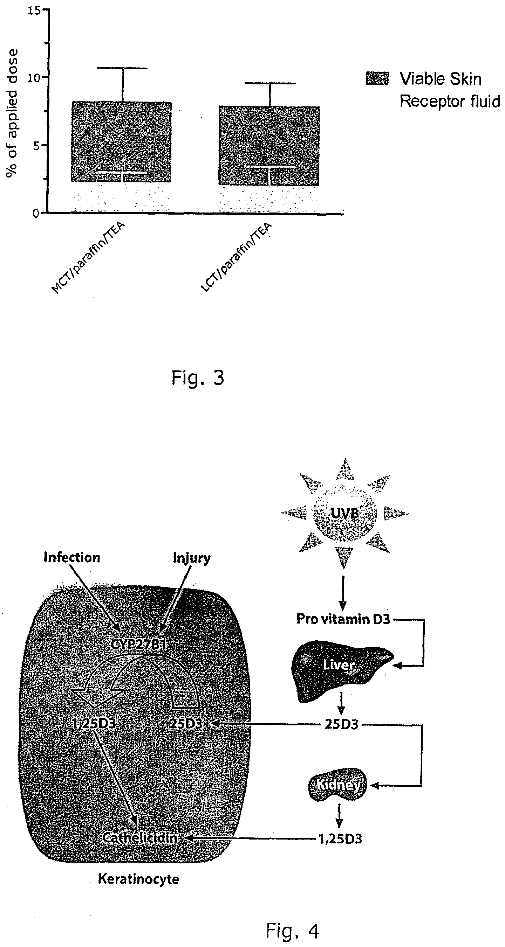

Fig. 3 is a graph showing the penetration into the skin and flux through the skin of calcipotriol from a composition of the invention as reported in Example 6 below. It appears from the figure that a significant amount of calcipotriol penetrated into the viable layers of the skin, whereas only a minor amount permeated through the skin into the receptor fluid.
Fig. 4 is a schematic representation of the activation of the gene encoding cathelicidin by vitamin D₃ in human keratinocytes. The mechanism of cathelicidin gene activation is used in a biological assay using reconstructed human epidermis (human keratinocytes cultured so as to form the epidermal layers characteristic of human skin) on which calcipotriol-containing compositions of the invention are applied to activate cathelicidin as described in detail in Example 7 below.

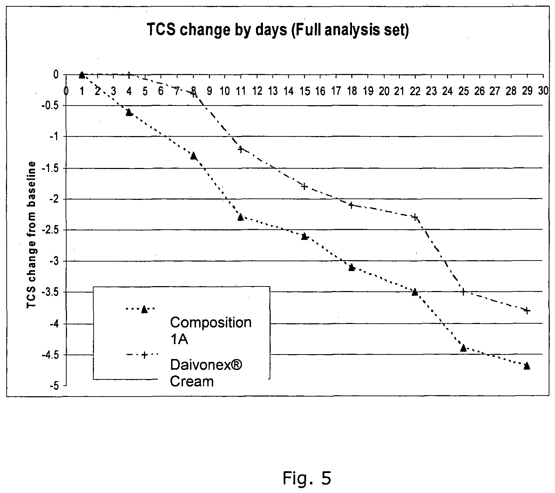

Fig. 5 is a graph showing the efficacy of a composition of the invention (Composition 1A) compared to Daivonex® cream on application on psoriatic plaques once daily for 29 days, determined as change in total clinical score (TCS).

### DETAILED DESCRIPTION OF THE INVENTION

### Definitions

In the present context, the term "non-ionic surfactant" is intended to indicate a surfactant comprising a hydrophilic and a hydrophobic portion in which the hydrophilic portion carries no charge but derives its surface activity from highly polar groups such as polyoxyethylene groups. For the present purpose, a hydrophilic surfactant is an oil-in-water surfactant with an HLB (hydrophilic-lipophilic balance) value of 10-18, and a lipophilic surfactant is a water-in-oil surfactant with an HLB value of 2-9, in particular 3-7.

The term "isotropic solvent mixture" is intended to indicate a mixture of solvents and/or surfactants which is capable of solubilizing or dissolving the vitamin D derivative or analogue and whose physical properties are independent of direction.

The term "vitamin D derivative" is intended to indicate a biologically active metabolite of vitamin D₃, such as calcitriol, or a precursor to such a metabolite, such as alfacalcidol.

The term "vitamin D analogue" is intended to indicate a synthetic compound comprising a vitamin D scaffold with sidechain modifications and/or modifications of the scaffold itself. The analogue exhibits a biological activity on the vitamin D receptor comparable to that of naturally occurring vitamin D compounds.

"Calcipotriol" is a vitamin D analogue of the formula

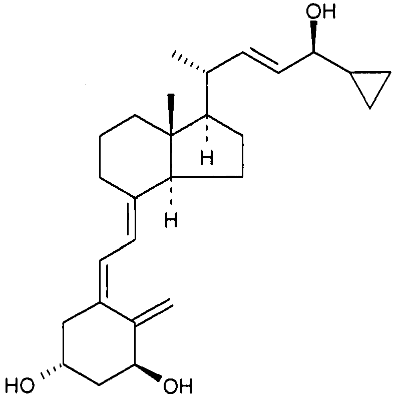

Calcipotriol has been found to exist in two crystalline forms, an anhydrate and a monohydrate. Calcipotriol monohydrate and its preparation are disclosed in WO 94/15912.

The term "storage stability" is intended to indicate that the composition exhibits chemical and physical stability characteristics that permit storage of the composition, at refrigeration or, preferably, room temperature for a sufficient period of time to make the composition commercially viable, such as at least 12 months, in particular at least 18 months, and preferably at least 2 years.

The term "chemical stability" or "chemically stable" is intended to indicate that no more than 10%, preferably no more than 6%, of the vitamin D derivative or analogue degrades over the shelf-life of the product, typically 2 years. An approximation of chemical stability at room temperature is obtained by subjecting the composition to accelerated stability studies at 40°C. If less than about 10% of the substance has degraded after 3 months at 40°C, this is usually taken to correspond to a shelf-life of 2 years at room temperature. In particular with respect to calcipotriol, "chemical stability" is intended to mean that the calcipotriol does not degrade significantly over time to 24-epi calcipotriol or other degradation products of calcipotriol in the finished pharmaceutical product.

The term "C₆₋₂₂ acylglyceride" is intended to indicate a triglyceride or a mixture of mono-and diglycerides or mono-, di- and triglycerides of C₆₋₂₂ fatty acids.

The term "medium chain triglycerides" is intended to indicate triglyceride esters of fatty acids with a chain length of 6-12 carbon atoms. A currently favoured example of such medium chain triglycerides is a mixture of caprylic (C₈) and capric (C₁₀) triglycerides, e.g. available under the trade name Miglyol 812.

The term "physical stability" or "physically stable" is intended to mean that the composition retains its macroscopic and microscopic appearance over the shelf-life of the product, e.g. that the vitamin D derivative or analogue does not precipitate from the solvent phase or that there is no phase separation of the solvent phase and carrier phase visible to the naked eye. Thus a composition wherein the isotropic solvent mixture is fully miscible with the lipid carrier and a composition wherein microscopic droplets of the isotropic solvent mixture are homogeneously distributed in the lipid carrier are both considered to be physically stable at this context.

The term "substantially anhydrous" is intended to mean that the content of free water in the lipophilic carrier or vehicle is less than about 2% by weight, preferably less than about 1% by weight, such as less than 0.5% by weight, of the carrier or vehicle.

The term "solubilization capacity" is intended to indicate the ability of the isotropic solvent mixture disclosed herein to dissolve a given substance, expressed as the amount required to effect complete solubilization of the substance.

The term "biological activity" is intended to mean the activity of a vitamin D derivative or analogue when applied to skin in a composition of the invention. The biological activity of compositions is determined in an *in vitro* assay measuring the activation of a target gene expressing the biomarker cathelicidin in a reconstructed human epidermis model involving cultured human keratinocytes, as described in detail in Example 7 below.

The term "skin penetration" is intended to mean the diffusion of the active ingredient into the different layers of the skin, i.e. the stratum corneum, epidermis and dermis.

The term "skin permeation" is intended to mean the flux of the active ingredient through the skin into the systemic circulation or, in case of *in vitro* studies such as those reported in Example 2 below, the receptor fluid of the Franz cell apparatus used in the experiment.

### Embodiments of the invention

The composition of the invention comprises a vitamin D derivative or analogue selected from the group consisting of calcipotriol, calcitriol, tacalcitol, maxacalcitol, paricalcitol and alfacalcidol. In a currently favoured embodiment, the composition comprises calcipotriol or calcipotriol monohydrate as the vitamin D analogue.

In an embodiment, the amount of the non-ionic surfactant in the isotropic mixture is from about 5% by weight to about 90% by weight, or from about 10% by weight to about 70% by weight, in particular from about 30% by weight to about 60% by weight, such as from about 40% by weight to about 50% by weight of the mixture.

The non-ionic surfactant is a hydrophilic surfactant with an HLB value of >9. The hydrophilic surfactant may for instance be a polyethylene glycol ester of a vegetable oil containing at least 20 mole of ethylene oxide groups/mole of glyceride, such esters being selected from the group consisting of polyoxyethylene castor oil derivatives, e.g. PEG 20, 30, 35, 38, 40, 50 or 60 castor oil or PEG 20, 25, 30, 40, 45, 50, 60 or 80 hydrogenated castor oil, PEG 20 or 60 corn glycerides, PEG 20 or 60 almond glycerides or PEG 40 palm kernel oil.

In an embodiment, the amount of the non-ionic co-surfactant in the isotropic mixture is from about 5% by weight to about 90% by weight, or from about 10% by weight to about 50% by weight, in particular from about 20% by weight to about 40% by weight, such as from about 25% by weight to about 30% by weight of the mixture.

The lipophilic surfactant may be selected from the group consisting of monoglyceride esters of C₆₋₂₂ fatty acids such as glyceryl monocaprylate, glyceryl monocaprate, glyceryl monostearate, glyceryl monobehenate, diglyceride esters of C₆₋₂₂ fatty acids such as glyceryl dilaurate, mono- and diglyceride esters of C₆₋₂₂ fatty acids such as caprylic/capric mono- and diglycerides or glyceryl mono- and diricinoleate, propylene glycol esters of C₆₋₂₂ fatty esters such as propylene glycol monocaprylate or propylene glycol monolaurate, dialkylene glycol monoalkyl ethers such as diethylene glycol monoethyl ether, polyglyceryl C₆₋₂₂ fatty acid esters such as polyglyceryl-3-diisostearate, polyethylene glycol esters of a triglyceride/vegetable oil containing 4-8 mole of ethylene oxide groups/mole of glyceride such as PEG-6 corn oil, PEG-6 almond oil, PEG-6 apricot kernel oil, PEG-6 olive oil, PEG-6 peanut oil, PEG-6 palm kernel oil or hydrogenated palm kernel oil, PEG-6 triolein or PEG-8 corn oil.

In the embodiment where both the surfactant and the co-surfactant are lipophilic surfactants, the surfactant and co-surfactant are preferably selected from different chemical surfactant classes.

In an embodiment, the amount of the C₆₋₂₂ acylglyceride in the isotropic solvent mixture is from about 5% by weight to about 90% by weight, or from about 10% by weight to about 70% by weight, e.g. from about 15% by weight to about 40% by weight such as from about 20% by weight to about 30% by weight of the mixture. The C₆₋₂₂ acylglyceride is a non-amphiphilic C₆₋₂₂ fatty acid glyceride selected from the group consisting of highly purified vegetable oils with an acid value of 0,1 or less, i.e. containing little or no acidic reacting substances such as free fatty acids, e.g. pharmaceutical grades of medium chain triglycerides, long chain triglycerides or castor oil, or an amphiphilic C₆₋₂₂ acylglyceride selected from the group consisting of caprylic/capric mono-and diglycerides and caprylic/capric mono-, di-and triglycerides. The ratio of first surfactant:second surfactant:C₆₋₂₂ acylglyceride may favourably be about 2:1:1 as this ratio may result in the formation of a monophasic system as shown in Figs. 1 and 2. Formation of a monophasic system is regarded as advantageous as such a system is generally physically stable, i.e. unlikely to result in phase separation. It is known from the literature that including a large amount of surfactant(s) in a dermal composition is likely to cause significant skin irritation. The isotropic solvent mixture included in the present composition is surprisingly effective to dissolve a sparingly soluble compound such as a vitamin D derivative or analogue. The high solubilization capacity where the mixture is more effective than the individual components of the mixture to dissolve the active ingredient makes it possible to use a lower amount of surfactants and consequently decrease the risk of skin irritation while retaining a high biological activity. Thus, unlike the compositions for oral use disclosed in the publications discussed above, the isotropic solvent mixture only constitutes a minor proportion of the composition, the lipid carrier and optionally other excipients making up the remainder of the composition. Thus, the isotropic solvent mixture may constitute about 1-20% by weight, such as about 5-15% by weight or about 8-12% by weight or about 9-11% by weight, e.g. about 10% by weight, of the composition.

In particularly favoured compositions of the invention, the C₆₋₂₂ acylglyceride is medium chain triglycerides, the surfactant is polyoxyl 40 hydrogenated castor oil, and the co-surfactant is caprylic/capric mono- and diglycerides, or the C₆₋₂₂ acylglyceride is long chain triglycerides, the surfactant is polyoxyl 40 hydrogenated castor oil, and the co-surfactant is caprylic/capric mono- and diglycerides.

The lipid carrier is a hydrocarbon or mixture of hydrocarbons with chain lengths ranging from C₅ to C₆₀. A frequently used ointment carrier is petrolatum, or white soft paraffin, which is composed of hydrocarbons of different chain lengths peaking at about C₄₀₋₄₄, or a mixture of petrolatum and liquid paraffin (consisting of hydrocarbons of different chain lengths peaking at C₂₈₋₄₀). While petrolatum provides occlusion of the treated skin surface, reducing transdermal loss of water and potentiating the therapeutic effect of the active ingredient in the composition, it tends to have a greasy and/or tacky feel which persists for quite some time after application, and it is not easily spreadable. It may therefore be preferred to employ paraffins consisting of hydrocarbons of a somewhat lower chain length, such as paraffins consisting of hydrocarbons with chain lengths peaking at C₁₄₋₁₆, C₁₈₋₂₂, C₂₀₋₂₂, C₂₀₋₂₆ or mixtures thereof (the hydrocarbon composition of the paraffins has been determined by gas chromatography). It has been found that such paraffins are more cosmetically acceptable in that they are less tacky and/or greasy on application and more easily spreadable. They are therefore expected to result in improved patient compliance. Suitable paraffins of this type, termed petrolatum jelly, are manufactured by Sonneborn and marketed under the trade name Sonnecone, e.g. Sonnecone CM, Sonnecone DM1, Sonnecone DM2 and Sonnecone HV. These paraffins are further disclosed and characterized in WO 2008/141078.

To impart a desired viscosity to the present composition, it may suitably include a lipophilic viscosity-increasing ingredient such as a wax. The wax may be a mineral wax composed of a mixture of high molecular weight hydrocarbons, e.g. saturated C₃₅₋₇₀ alkanes, such as microcrystalline wax. Alternatively, the wax may be a vegetable or animal wax, e.g. esters of C₁₄₋₃₂ fatty acids and C₁₄₋₃₂ fatty alcohols, such as beeswax. The amount of viscosity-increasing ingredient may vary according to the viscosifying power of the ingredient, but may typically be in the range of about 1-20% by weight of the composition. When the viscosity-increasing ingredient is microcrystalline wax it is typically present in an amount in the range of about 5-15% by weight, e.g. about 10% by weight, of the composition.

The composition may additionally comprise an emollient which may act to soften the thickened epidermis of the psoriatic plaques. A suitable emollient for inclusion in the present composition may be a silicone wax or a volatile silicone oil as the presence of silicone has additionally been found to aid penetration of calcipotriol into the skin. Compositions including silicone oil have also been found to result in less skin irritation. Suitable silicone oils for inclusion in the present composition may be selected from cyclomethicone, dimethicone. The amount of silicone oil included in the present composition is typically in the range of from about 1 to about 10% by weight, e.g. about 5% by weight, of the composition.

In Daivonex^{®} ointment, the presence of propylene glycol is believed to be a major contributor to the skin irritation experienced by many patients. However, it has been found that calcipotriol may in itself be mildly irritative in some patients (A. Fullerton and J. Serup, Br. J. Dermatol. 137, 1997, pp. 234-240 and A. Fullerton et al., Br. J. Dermatol. 138, 1998, pp. 259-265). It may therefore be advantageous to include an anti-irritant compound in the present composition, such as glycerol, butylene glycol, sorbitol, sucrose, saccharin, menthol or nicotinamide. Glycerol has been described as a substance that is capable of protecting the skin against irritative substances (J. Bettinger et al., Dermatology 197, 1998, pp. 18-24) and has been found by us to reduce the release of IL-1α in a dose-dependent manner: thus, it has been found that the presence of 15% by weight of glycerol in a calcipotriol ointment results in a significantly lower level of release of IL-1α than does the inclusion of 10% by weight of glycerol which, in turn, results in a significantly lower level of IL-1α release than does the inclusion of 5% by weight of glycerol.

However, in addition to the anti-irritative effect, it has surprisingly been found that glycerol is capable of potentiating the biological activity of calcipotriol in that the expression of cathelicidin (in the assay described in Example 7 below) has been found to be increased with a low amount of glycerol in the composition (i.e. more cathelicidin is expressed when the amount of glycerol is 5% by weight than when the amount of glycerol is 10% or 15%, respectively). This implies that with respect to inclusion of glycerol a balance has to be struck between a favourable anti-irritative effect and a favourable potentiating effect. We have found that the inclusion of about 5-10% by weight of glycerol in the present composition results in a significant anti-irritative effect as well as a significant potentiation of the biological activity of calcipotriol.

Calcipotriol is known to be a substance which is extremely sensitive to acid conditions (pH below about 7.0 in aqueous compositions or acidic reacting substances in nonaqueous compositions) which contribute to the rapid degradation of calcipotriol. To ensure an adequate chemical stability of the substance throughout the shelf-life of the composition, it may be advisable to include a compound capable of neutralizing acidic impurities which may be present in one or more of the excipients of the composition and which are detrimental to the chemical stability of calcipotriol. The acid neutralizing compound may favourably be selected from a buffer such as a phosphate buffer which may be included in an amount of about 0.025-0.1% by weight of the composition. The acid neutralizing compound may also be a tertiary amine such as triethanolamine, trometamol, monoethanolamine or diethanolamine, which may be included in the composition in an amount of about 0.1-2% by weight.

In a specific embodiment, the present composition comprises
0.003-0.008% w/w of calcipotriol monohydrate
2-3% w/w medium or long chain triglycerides
2-3% w/w caprylic/capric mono- and diglycerides
4-6% w/w PEG 40 hydrogenated castor oil
0.5-1.5% w/w triethanolamine
85-95% w/w paraffin carrier

Disclosed is a composition which comprises
0.003-0.008% w/w of calcipotriol monohydrate
0.5-1.5% w/w of caprylic/capric mono-, di- and triglycerides
10-20% w/w PEG-6 corn oil
5-15% w/w polyglyceryl-3-diisostearate, diethylene glycol monoethyl ether or propylene glycol monolaurate or monocaprylate
0.5-1.5% w/w triethanolamine
75-80% w/w paraffin carrier

The present composition may also comprise other components commonly used in dermal formulations, e.g. antioxidants (e.g. alpha-tocopherol), preservatives, sodium edetate, pigments, skin soothing agents, skin healing agents and skin conditioning agents such as urea, allantoin or bisabolol, cf. CTFA Cosmetic Ingredients Handbook, 2nd Ed., 1992. The composition of the invention may be used in the treatment of psoriasis, sebopsoriasis, pustulosis palmoplantaris, dermatitis, ichtyosis, rosacea and acne and related skin diseases by topically administering an effective amount of a composition according to the invention to a patient in need of such treatment. Said method preferably comprises topical administration once or twice a day of a therapeutically sufficient dosage of said composition. To that end, the composition according to the invention preferably contains about 0.001-0.5 mg/g, preferably about 0.002-0.25 mg/g, in particular 0.005-0.05 mg/g, of the vitamin D derivative or analogue. It is envisaged that the present composition may advantageously been used for maintenance treatment of these dermal diseases, i.e. continued treatment after the diseappearance of visible symptoms to delay the recurrence of symptoms.

To provide a more effective treatment of psoriasis and other dermal conditions in the acute phase, it may be desirable to include one or more additional therapeutically active ingredients in the composition. Examples of such additional active ingredients include, but are not limited to, anti-inflammatory drugs such as corticosteroids, such as betamethasone and esters thereof, e.g. the valerate or dipropionate ester, clobetasol or esters thereof, such as the propionate, hydrocortisone or esters thereof, such as the acetate; non-steroidal anti-inflammatory drugs such as naproxen, indomethacin, diclofenac, ibuprofen, dexibuprofen, ketoprofen, flurbiprofen, piroxicam, tenoxicam, lornoxicam or nabumeton, phosphodiesterase 4 inhibitors (e.g. the PDE4 inhibitors disclosed in WO 2008/077404, WO 2008/104175, WO 2008/128538 or WO 2010/069322) or p38 MAP kinase inhibitors (e.g. the p38 MAP kinase inhibitors disclosed in WO 2005/009940 or WO 2006/063585).

The invention is further illustrated by the following examples which are not in any way intended to limit the scope of the invention as claimed.

### Example 1

### Compositions of the invention

| Ingredient (mg/g) | Comp. 1A | Comp. 1B |
|---|---|---|
| calcipotriol monohydrate | 0.05 | 0.05 |
| medium chain triglycerides (Miglyol 812) | 25 | |
| long chain triglycerides (sesame oil) | | 25 |
| caprylic/capric glycerides (Akoline MCM) | 27 | |
| glycerol monooleate 40 (Peceol) | | 27 |
| polyoxyl 40 hydrogenated castor oil (Cremophor RH 40) | 48 | 48 |
| white soft paraffin | 890 | 890 |
| triethanolamine | 10 | 10 |

Composition 1A was prepared by mixing the medium chain triglycerides, caprylic/capric glycerides and polyoxyl 40 hydrogenated castor oil and stirring the mixture for 15 min. at 50°C with a magnetic stirrer. The calcipotriol monohydrate was dissolved in the mixture at 40°C using a magnetic stirrer for 15 min. White soft paraffin was melted at 80°C, and triethanolamine was dissolved in the melted paraffin. The three-component surfactant-solvent mixture containing calcipotriol was added to the melted paraffin and whisked until the ointment mixture was homogenous. The homogenized ointment was cooled to 30°C with stirring and filled into 15 g aluminium tubes. Composition 1B was prepared in a similar fashion with the exception that glycerol monooleate 40 was used as the co-surfactant instead of caprylic/capric glycerides.

The compositions were tested for chemical stability at 40°C for 3 months. The results showed a satisfactory stability of calcipotriol under the test conditions.

### Example 2

### Compositions (not according to the invention)

| Ingredient (mg/g) | Comp.2A | Comp. 2B | Comp.2C | Comp.2D | Comp. 2E | Comp.2F |
|---|---|---|---|---|---|---|
| calcipotriol monohydrate | 0.0522 | 0.0522 | 0.0522 | 0.0522 | 0.0522 | 0.0522 |
| lauroyl macrogol-6-glycerides (Labrafil M2130 CS) | 100 | 150 | 170 | 134 | 134 | 134 |
| polyglyceryl-3-diisostearate (Plurol Diisostearique) | 100 | | | | | |
| linoleyl macrogol-6-glyceride (Labrafil M2125CS) | | 50 | | | | |
| diethylene glycol monoethyl ether(Transcutol P) | | | 30 | | | |
| propylene glycol monolaurate (Lauroglycol 90) | | | | 66 | | |
| propylene glycol monocaprylate (Capryol 90) | | | | | 66 | |
| propylene glycol monocaprylate (Capryol 90) | | | | | | 66 |
| glycerol monocaprylocaprate (IMWITOR 742) | 10 | 10 | 10 | 10 | 10 | 10 |
| white soft paraffin | 780 | 780 | 780 | 780 | 780 | 790 |
| triethanolamine | 10 | 10 | 10 | 10 | 10 | 10 |

Compositions 2A-2F were prepared in a similar fashion as composition 1A, but with appropriate substitution of the surfactant, co-surfactant and solvent as indicated in the table above.

The compositions were tested for chemical stability at 40°C for 3 months. The results showed a satisfactory stability of calcipotriol under the test conditions.

### Example 3

### Compositions of the invention

| Ingredient (mg/g) | 3A | 3B | 3C | 3D | 3E | 3F | 3G | 3H |
|---|---|---|---|---|---|---|---|---|
| calcipotriol monohydrate | 0.0522 | 0.0522 | 0.0522 | 0.0522 | 0.0522 | 0.0522 | 0.0522 | 0.0522 |
| Medium chain triglycerides (Miglyol 812) | 15 | 32 | 15 | 40 | 30 | 60 | 15 | 80 |
| Caprylic/capric glycerides (Akoline MCM) | 15 | 13 | 40 | 20 | 40 | 20 | 70 | 10 |
| Polyoxyl 40 hydrogenated castor oil (Cremophor RH40) | 70 | 55 | 45 | 40 | 30 | 20 | 15 | 10 |
| white soft paraffin | 890 | 890 | 890 | 890 | 890 | 890 | 890 | 890 |
| triethanolamine | 10 | 10 | 10 | 10 | 10 | 10 | 10 | 10 |

Compositions 3A-3H were prepared as described in Example 1, but with the appropriate amounts of solvent, surfactant and co-surfactant shown in the table above.

### Example 4

### Compositions of the invention

| Ingredient (mg/g) | 4A | 4B | 4C | 4D | 4E | 4F |
|---|---|---|---|---|---|---|
| calcipotriol monohydrate | 0.0522 | 0.0522 | 0.0522 | 0.0522 | 0.0522 | 0.0522 |
| Medium chain triglycerides (Miglyol 812) | 25 | 25 | 25 | 25 | 25 | 25 |
| Caprylic/capric glycerides (Akoline MCM) | 27 | 27 | 27 | 27 | 27 | 27 |
| Polyoxyl 35 castor oil (Cremophor EL) | 48 | | | | | |
| PEG-20 hydrogenated castor oil (Nikkol HCO 20) | | 48 | | | | |
| PEG-30 hydrogenated castor oil (Nikkol HCO 30) | | | 48 | | | |
| PEG-50 hydrogenated castor oil (Nikkol HCO 50) | | | | 48 | | |
| PEG-60 hydrogenated castor oil (Taqat R 60) | | | | | 48 | |
| PEG-80 hydrogenated castor oil (Nikkol HCO 80) | | | | | | 48 |
| White soft paraffin | 890 | 890 | 890 | 890 | 890 | 890 |
| triethanolamine | 10 | 10 | 10 | 10 | 10 | 10 |

| Ingredient (mg/g) | 4G | 4H | 41 | 4J | 4K | 4L |
|---|---|---|---|---|---|---|
| calcipotriol monohydrate | 0.0522 | 0.0522 | 0.0522 | 0.0522 | 0.0522 | 0.0522 |
| Medium chain triglycerides (Miglyol 812) | 30 | 30 | 30 | 30 | 30 | 30 |
| Caprylic/capric glycerides (Akoline MCM) | 40 | 40 | 40 | 40 | 40 | 40 |
| Polyoxyl 35 castor oil (Cremophor EL) | 30 | | | | | |
| PEG-20 hydrogenated castor oil (Nikkol HCO 20) | | 30 | | | | |

| | | | | | | |
|---|---|---|---|---|---|---|
| PEG-30 hydrogenated castor oil (Nikkol HCO 30) | | | 30 | | | |
| PEG-50 hydrogenated castor oil (Nikkol HCO 50) | | | | 30 | | |
| PEG-60 hydrogenated castor oil (Tagat R 60) | | | | | 30 | |
| PEG-80 hydrogenated castor oil (Nikkol HCO 80) | | | | | | 30 |
| White soft paraffin | 890 | 890 | 890 | 890 | 890 | 890 |
| triethanolamine | 10 | 10 | 10 | 10 | 10 | 10 |

Compositions 4A-4L were prepared as described in Example 1, but with the appropriate amounts of solvent, surfactant and co-surfactant shown in the table above.

### Example 5

### Compositions of the invention

| Ingredient (mg/g) | 5A | 5B | 5C | 5D | 5E | 5F | 5G |
|---|---|---|---|---|---|---|---|
| calcipotriol monohydrate | 0.0522 | 0.0522 | 0.0522 | 0.0522 | 0.0522 | 0.0522 | 0.0522 |
| Medium chain triglycerides (Miglyol 812) | 15 | 32 | 15 | 40 | 30 | 60 | 15 |
| Caprylic/capric glycerides (Akoline MCM) | 15 | 13 | 40 | 20 | 40 | 20 | 70 |
| Polyoxyl 40 hydrogenated castor oil (Cremophor RH40) | 48 | 48 | 48 | 48 | 48 | 48 | 48 |
| Triethanolamine | 10 | 10 | 10 | 10 | 10 | 10 | 10 |
| Microcrystalline wax | | | | | 50 | 100 | 150 |
| Petrolatum jelly white (Sonnecone CM) | ad 1g | | | | | | |
| Petrolatum jelly white (Sonnecone DM1) | | ad 1 g | | | ad 1 g | ad 1 g | ad 1 g |
| Petrolatum jelly white (Sonnecone DM2) | | | ad 1 g | | | | |
| Petrolatum jelly white (Sonnecone HV) | | | | ad 1 g | | | |

Compositions 5A-5G were prepared as described in Example 1, but with the appropriate amounts of Petrolatum jelly white shown in the table above.

### Example 6

### Penetration studies

To investigate the skin penetration and permeation of calcipotriol from compositions of the invention, a skin diffusion experiment was conducted. Full thickness skin from pig ears was used in the study. The ears were kept frozen at -18°C before use. On the day prior to the experiment the ears were placed in a refrigerator (5±3°C) for slow defrosting. On the day of the experiment, the hairs were removed using a veterinary hair trimmer. The skin was cleaned for subcutaneous fat using a scalpel and two pieces of skin were cut from each ear and mounted on Franz diffusion cells in a balanced order.

Static Franz-type diffusion cells with an available diffusion area of 3.14 cm² and receptor volumes ranging from 8.6 to 11.1 ml were used in substantially the manner described by T.J. Franz, "The finite dose technique as a valid in vitro model for the study of percutaneous absorption in man", in Current Problems in Dermatology, 1978, J.W.H. Mall (Ed.), Karger, Basel, pp. 58-68. The specific volume was measured and registered for each cell. A magnetic bar was placed in the receptor compartment of each cell. After mounting the skin, physiological saline (35°C) was filled into each receptor chamber for hydration of the skin. The cells were placed in a thermally controlled water bath which was placed on a magnetic stirrer set at 400 rpm. The circulating water in the water baths was kept at 35±1°C resulting in a temperature of about 32°C on the skin surface. After one hour the saline was replaced by receptor medium, 0.04 M isotonic phosphate buffer, pH 7.4 (35°C), containing 4% bovine serum albumin. Sink conditions were maintained at all times during the period of the study, i.e. the concentration of the active compounds in the receptor medium was below 10% of the solubility of the compounds in the medium.

The *in vitro* skin permeation of each test composition was tested in 6 replicates (i.e. n=6). Each test composition was applied to the skin membrane at 0 hours in an intended dose of 4 mg/cm². A glass spatula was used for the application, and the residual amount of the composition was determined so as to give the amount of the composition actually applied on the skin.

The skin penetration experiment was allowed to proceed for 21 hours. Samples were then collected from the following compartments:
The stratum corneum was collected by tape stripping 10 times using D-Squame^{®} tape (diameter 22 mm, CuDerm Corp., Dallas, Texas, USA). Each tape strip is applied to the test area using a standard pressure for 5 seconds and removed from the test area in one gentle, continuous move. For each repeated strop, the direction of tearing off was varied. The viable epidermis and dermis was then sampled from the skin in a similar fashion.

Samples (1 ml) of the receptor fluid remaining in the diffusion cell were collected and analysed.

The concentration of calcipotriol in the samples were determined by LC mass spectrometry.

The results appear from Figure 3 below which shows the amount of calcipotriol found in viable skin (dermis and epidermis) and receptor fluid in % of the applied dose. Very little of the applied calcipotriol was found in the receptor fluid, suggesting that on application of the present compositions *in vivo,* only a minor amount of the active ingredient will permeate through the skin into the systemic circulation, thus minimizing the risk of systemic adverse effects.

### Example 7

### Biological activity of the compositions

As shown in figure 4 below, cathelicidin is an antimicrobial peptide expressed in human keratinocytes. The expression of cathelicidin is strongly induced on infection of the skin or disruption of the skin barrier. In psoriasis, the level of cathelicidin is increased in lesional skin of psoriasis patients. It has been found that the expression of the gene encoding cathelicidin may be induced by vitamin D₃ or vitamin D analogues such as calcipotriol (cf. TT Wang et al, J. Immunol. 173(5), 2004, pp. 2909-2912; J Schauber et al., Immunology 118(4), 2006, pp. 509-519; Schauber and Gallo, J. Allergy Clin Immunol 122, 2008, pp. 261-266; M. Peric et al., PloS One 4(7), July 22, 2009, e6340) through binding to the vitamin D receptor. This finding has been utilized to develop an assay in which the uptake and biological activity of calcipotriol in human keratinocytes from the tested compositions has been determined by measuring the level of induction of the gene encoding cathelicidin.

In the assay, composition 1A prepared as described in Example 1 above was applied topically in triplicate on reconstructed human epidermis consisting of normal human keratinocytes cultured for 12 days on 0.5 cm² polycarbonate filters (available from SkinEthic^{®} Laboratories, Nice, France) in an amount of 10 µl. The tissue was treated for two days followed by separation of the epidermis from the polycarbonate filter and snap-frozen in liquid nitrogen. RNA was extracted from the cells and cDNA synthesized by conventional procedures. Quantitative real-time PCR (qPCR) was then performed using the following assays from Applied Biosystems: CAMP Hs0018038_ml and GAPDH Hs99999905_ml. The expression levels of cathelicidin were normalized to GAPDH and a relative quantification was made by comparison with Daivonex^{®} ointment.

Three consecutive experiments were carried out in this manner. The results from the first two experiments showed a 6.2 and 5.7 fold increase, respectively, in the biological activation of cathelicidin relative to that obtained with Daivonex^{®} ointment, while the results of the third experiment showed a 12.9 fold increase.

When Composition 1B (prepared as described in Example 1 above) was tested in this assay, the results from the two experiments showed a 2.7 and 1.5 fold increase, respectively, in the biological activation of cathelicidin relative to that obtained with Daivonex^{®} ointment.

In table 1 below are listed the results obtained when compositions 3A, 3H, 3G and 4A-F were tested in this assay:

**Table 1 Biological activity of compounds of the present invention.**

| Composition | Fold increase in biological activation of cathelicidin |
|---|---|
| 3A | 1,50 |
| 3H | 2,40 |
| 3G | 5,35 |
| 4A | 3,17 |
| 4B | 0,95 |
| 4C | 1,69 |
| 4D | 1,63 |
| 4E | 1,25 |
| 4F | 0,91 |

### Example 8

### Local tolerance study in minipigs

The local tolerability of composition 1A of Example 1 was assessed when administered daily by dermal application to minipigs for 4 weeks. Each day the animals were exposed to the test items for 8 hours.

The study was conducted in 10 female Göttingen SPF minipigs. Each animal had 6 application sites and received a volume of 250 mg test formulation per application site. Clinical signs were recorded daily and skin reactions at the application sites were scored once daily prior to start of dosing and, furthermore, on the day of necropsy in relation to erythema and oedema. Food consumption was recorded daily and the body weight weekly. At the end of the treatment period a gross necropsy was performed on all animals and skin samples were collected from histopathological examination.

The results show that no adverse treatment-related clinical signs were observed during the study. No scores in relation to erythema were observed for composition 1A. The results imply that compositions of the invention will be well tolerated in human patients as well.

### Example 9

### Plaque studies in psoriasis patients

Compound 1A was tested in a psoriasis plaque test. The study consisted of a screening visit, a wash-out period if needed, a treatment period of 29 days, and, if applicable, a follow-up visit. Within 15 days before treatment a screening visit for study eligibility of the subjects took place. Prior to Day 1 (Visit 2) a washout period (up to 15 days) was completed if the subject was treated with anti-psoriatic treatments or other relevant medication. Treatment products, the investigational product and the reference product (Daivonex^{®} cream) were given once daily 6 days a week (except Sundays) for four (4) weeks. The subjects received study medication on test sites of 2 cm diameter selected on predetermined psoriasis lesions. Twice a week during the treatment phase, clinical assessments were performed. Further, ultrasound measurements of skin thickness were performed at Day 1 (baseline), three times during the study and at end of treatment period.

The primary response criterion was the absolute change in Total Clinical Score (TCS) of clinical symptoms (sum of erythema, scaling and infiltration) at the end of the treatment period compared to baseline. The change in total lesion thickness measured by ultrasound at end of treatment and at each assessment compared to baseline was also determined.

The results shown in Fig. 5 indicate that the improvement in TCS is more pronounced and has a faster onset when psoriatic plaques are treated with Composition 1A than when they are treated with Daivonex® cream.

### Example 10

### Compositions of the invention

| Ingredient (mg/g) | Comp. 6A |
|---|---|
| calcipotriol monohydrate | 0.05 |
| A PDE4 inhibitor compound | 2.5 |
| medium chain triglycerides (Miglyol 812) | 25 |
| caprylic/capric glycerides (Akoline MCM) | 27 |
| glycerol monooleate 40 (Peceol) | |
| polyoxyl 40 hydrogenated castor oil (Cremophor RH 40) | 48 |
| white soft paraffin | 887.5 |
| triethanolamine | 10 |

| Ingredient (mg/g) | Comp. 6B |
|---|---|
| calcipotriol monohydrate | 0.0522 |
| A PDE4 inhibitor compound | 2.5 |
| Medium chain triglycerides (Miglyol 812) | 25 |
| Caprylic/capric glycerides (Akoline MCM) | 27 |
| Polyoxyl 40 hydrogenated castor oil (Cremophor RH40) | 48 |
| Triethanolamine | 10 |
| Microcrystalline wax | 100 |
| Petrolatum jelly white (Sonnecone DM1) | ad 1 g |

| Ingredient (mg/g) | Comp. 6C |
|---|---|
| calcipotriol monohydrate | 0.0522 |
| A PDE4 inhibitor compound | 2.5 |
| Medium chain triglycerides (Miglyol 812) | 15 |
| Caprylic/capric glycerides (Akoline MCM) | 70 |
| Polyoxyl 40 hydrogenated castor oil (Cremophor RH40) | 15 |
| white soft paraffin | 887.5 |
| triethanolamine | 10 |

Compositions 6A, 6B and 6C were prepared as disclosed in Example 1, except for the addition of the PDE4 compound.

## Claims

1. A substantially anhydrous pharmaceutical composition for cutaneous application comprising 1-20% by weight of an isotropic solvent mixture of
(a) a hydrophilic non-ionic surfactant which is a polyethylene glycol ester of a vegetable oil containing at least 20 mole of ethylene oxide groups/mole of glyceride;
(b) a lipophilic non-ionic co-surfactant which is selected from the group consisting of monoglyceride esters of C₆₋₂₂ fatty acids, diglyceride esters of C₆₋₂₂ fatty acids, mono-and diglyceride esters of C₆₋₂₂ fatty acids, propylene glycol esters of C₆₋₂₂ fatty acids, dialkylene glycol monoalkyl ethers, or polyethylene glycol esters of a triglyceride/vegetable oil containing 4-8 mole of ethylene oxide groups/mole of glyceride;
(c) a C₆₋₂₂ acylglyceride which is a non-amphiphilic C₆₋₂₂ acylglyceride selected from the group consisting of highly purified vegetable oils such as medium chain triglycerides, long chain triglycerides or castor oil;
said isotropic solvent mixture being capable of forming a microemulsion in the presence of an excess of water;
the composition further comprising a vitamin D derivative or analogue dissolved or solubilized in said isotropic solvent mixture, the vitamin D derivative or analogue being selected from the group consisting of calcipotriol, calcitriol, tacalcitol, maxacalcitol, paricalcitol and alfacalcidol, and
a pharmaceutically acceptable, substantially anhydrous lipid carrier which is a paraffin selected from hydrocarbons with chain lengths from C₅ to C₆₀.

2. A composition according to claim 1, wherein the vitamin D analogue is calcipotriol or calcipotriol monohydrate.

3. A composition according to any one of claims 1-2, wherein the amount of the hydrophilic surfactant in the isotropic solvent mixture is from 10% by weight to 70% by weight, in particular from 30% by weight to 60% by weight, such as from 40% by weight to 50% by weight of the mixture.

4. A composition according to claim 3, wherein the polyethylene glycol ester of a vegetable oil containing at least 20 mole of ethylene oxide groups/mole of glyceride is selected from the group consisting of polyoxyethylene castor oil derivatives, e.g. PEG 20, 30, 35, 38, 40, 50 or 60 castor oil or PEG 20, 25, 30, 40, 45, 50, 60 or 80 hydrogenated castor oil, PEG 20 or 60 corn glycerides, PEG 20 or 60 almond glycerides or PEG 40 palm kernel oil.

5. A composition according to any one of claims 1-4, wherein the amount of the lipophilic non-ionic co-surfactant in the isotropic solvent mixture is from 10% by weight to 50% by weight, in particular from 20% by weight to 40% by weight, such as from 25% by weight to 30% by weight of the mixture.

6. A composition according to claim 1, wherein the monoglyceride esters of C₆₋₂₂ fatty acids are glyceryl monocaprylate, glyceryl monocaprate, glyceryl monostearate, glyceryl monobehenate, or wherein the diglyceride esters of C₆₋₂₂ fatty acids are glyceryl dilaurate, or wherein the mono- and diglyceride esters of C₆₋₂₂ fatty acids are caprylic/capric mono- and diglycerides or glyceryl mono- and diricinoleate, or wherein the propylene glycol esters of C₆₋₂₂ fatty acids are propylene glycol monocaprylate or propylene glycol monolaurate, or wherein the dialkylene glycol monoalkyl ethers are diethylene glycol monoethyl ether, or wherein the polyethylene glycol esters of a triglyceride/vegetable oil containing 4-8 mole of ethylene oxide groups/mole of glyceride are PEG-6 corn oil, PEG-6 almond oil, PEG-6 apricot kernel oil, PEG-6 olive oil, PEG-6 peanut oil, PEG-6 palm kernel oil or hydrogenated palm kernel oil, PEG-6 triolein or PEG-8 corn oil.

7. A composition according to any one of claims 1-6, wherein the amount of C₆₋₂₂ acylglyceride in the isotropic solvent mixture is from 10% by weight to 70% by weight, e.g. from 15% by weight to 40% by weight, such as from 20% by weight to 30% by weight of the mixture.

8. A composition according to any one of claims 1-7 wherein the ratio of hydrophilic surfactant:lipophilic co-surfactant:C₆₋₂₂ acylglyceride is 2:1:1.

9. A composition according to any one of claims 1-8, wherein the isotropic solvent mixture of claim 1 constitutes 5-15% by weight or 8-12% by weight or 9-11% by weight, e.g. 10% by weight, of the composition.

10. A composition according to any one of claims 1-9, wherein the C₆₋₂₂ acylglyceride is medium chain triglycerides, the surfactant is polyoxyl 40 hydrogenated castor oil, and the co-surfactant is caprylic/capric mono- and diglycerides, or wherein the C₆₋₂₂ acylglyceride is long chain triglycerides, the surfactant is polyoxyl 40 hydrogenated castor oil, and the co-surfactant is caprylic/capric mono- and diglycerides.

11. A composition according to any one of claims 1-10, wherein the lipid carrier comprises at least one paraffin selected from paraffins consisting of hydrocarbons with chain lengths from C₅ to C₆₀, the chain lengths peaking at C₁₄₋₁₆, C₁₈₋₂₂, C₂₀₋₂₂, C₂₀₋₂₆, C₂₈₋₄₀, and C₄₀₋₄₄ (as determined by gas chromatography) or mixtures thereof.

12. A composition according to any one of claims 1-11, further comprising a viscosity-increasing ingredient.

13. A composition according to claim 12, wherein the viscosity-increasing ingredient is a wax.

14. A composition according to any one of claims 1-13, further comprising a silicone wax or a volatile silicone oil.

15. A composition according to claim 14, wherein the volatile silicone oil is cyclomethicone or dimethicone.

16. A composition according to any one of claims 1-15, further comprising an anti-irritant compound.

17. A composition according to claim 16, wherein the anti-irritant compound is glycerol, butylene glycol, sorbitol, sucrose, saccharin, menthol or nicotinamide.

18. A composition according to claim 17 comprising 5-10% by weight of glycerol.

19. A composition according to any one of claims 1-18, further comprising a compound capable of neutralizing acidic impurities detrimental to the chemical stability of the vitamin D derivative or analogue in the composition.

20. A composition according to claim 19, wherein said compound is an amine such as triethanol amine, trometamol, monoethanolamine or diethanolamine.

21. A composition according to any one of claims 1-20 comprising 0.001-0.5 mg/g, preferably 0.002-0.25 mg/g, in particular 0.005-0.05 mg/g, of the vitamin D derivative or analogue.

22. A composition according to any one of claims 1-21 comprising
0.003-0.008% w/w of calcipotriol monohydrate
2-3% w/w medium or long chain triglycerides
2-3% w/w caprylic/capric mono- and diglycerides
4-6% w/w PEG 40 hydrogenated castor oil
0.5-1.5% w/w triethanolamine
85-95% w/w paraffin carrier

23. A composition according to any one of claims 1-22, further comprising one or more additional therapeutically active ingredients.

24. A composition according to claim 23, wherein such additional active ingredients are selected from the group consisting of anti-inflammatory drugs such as corticosteroids, such as betamethasone and esters thereof, e.g. the valerate or dipropionate ester, clobetasol or esters thereof, such as the propionate, hydrocortisone or esters thereof, such as the acetate; non-steroidal anti-inflammatory drugs such as naproxen, indomethacin, diclofenac, ibuprofen, dexibuprofen, ketoprofen, flurbiprofen, piroxicam, tenoxicam, lornoxicam or nabumeton, phosphodiesterase 4 inhibitors or p38 MAP kinase inhibitors.

25. A composition according to claim 24, wherein the additional active ingredient is betamethasone or esters thereof, e.g. the valerate or dipropionate esters.

26. A composition according to claim 24, wherein the additional active ingredient is a phosphodiesterase 4 inhibitor.

27. A composition according to any one of claims 1-26 for use in the treatment of a dermal disease or condition.

28. The composition of claim 27, for use in the treatment of a dermal disease or condition selected from the group consisting of psoriasis, sebopsoriasis, pustulosis palmoplantaris, dermatitis, ichtyosis, rosacea or acne.

## Patentansprüche

1. Im Wesentlichen wasserfreie pharmazeutische Zusammensetzung für die kutane Anwendung, umfassend 1 bis 20 Gew.-% eines isotropen Lösungsmittelgemischs aus:
(a) einem hydrophilen, nichtionischen Tensid, bei dem es sich um einen Polyethylenglykolester eines Pflanzenöls handelt, wenigstens 20 Mol Ethylenoxidgruppen/Mol Glycerid enthaltend;
(b) einem lipophilen, nichtionischen Cotensid, das ausgewählt ist aus der Gruppe bestehend aus Monoglyceridestern von C₆₋₂₂-Fettsäuren, Diglyceridestern von C₆₋₂₂-Fettsäuren, Mono- und Diglyceridestern von C₆₋₂₂-Fettsäuren, Propylenglykolestern von C₆₋₂₂-Fettsäuren, Dialkylenglykolmonoalkylethern oder Polyethylenglykolestern eines Triglycerids/Pflanzenöls, 4-8 Mol Ethylenoxidgruppen/Mol Glycerid enthaltend;
(c) einem von C₆₋₂₂-Acylglyerid, das ein nicht-amphiphiles C₆₋₂₂-Acylglyerid ist, das ausgewählt ist aus der Gruppe bestehend aus hoch aufgereinigten Pflanzenölen, wie etwa mittelkettigen Triglyceriden, langkettigen Triglyceriden oder Rizinusöl;
wobei das isotrope Lösungsmittelgemisch in Gegenwart eines Wasserüberschusses eine Mikroemulsion bilden kann;
wobei die Zusammensetzung ferner ein Vitamin-D-Derivat oder -Analog umfassen kann, das in dem isotropen Lösungsmittelgemisch aufgelöst oder löslich gemacht ist, wobei das Vitamin-D-Derivat oder -Analog ausgewählt ist aus der Gruppe bestehend aus Calcipotriol, Calcitriol, Tacalcitol, Maxacalcitol, Paricalcitol und Alfacalcidol; und
einem pharmazeutisch akzeptablen, im Wesentlichen wasserfreien Lipidträger, bei dem es sich um ein Paraffin handelt, ausgewählt aus Kohlenwasserstoffen mit Kettenlängen von C₅ bis C₆₀.

2. Zusammensetzung nach Anspruch 1, wobei das Vitamin-D-Analog Calcipotriol oder Calcipotriol-Monohydrat ist.

3. Zusammensetzung nach einem der Ansprüche 1 bis 2, wobei die Menge des hydrophilen Tensids in dem isotropen Lösungsmittelgemisch zwischen 10 und 70 Gew.-% liegt, insbesondere zwischen 30 und 60 Gew.-%, wie etwa zwischen 40 und 50 Gew.-% des Gemischs.

4. Zusammensetzung nach Anspruch 3, wobei der Polyethylenglykolester eines Pflanzenöls, wenigstens 20 Mol Ethylenoxidgruppen/Mol Glycerid enthaltend, ausgewählt ist aus der Gruppe bestehend aus Polyoxyethylen-Rizinusölderivaten, z.B. PEG 20, 30, 35, 38, 40, 50 oder 60 Rizinusöl oder PEG 20, 25,30, 40, 45, 50, 60 oder 80 hydriertes Rizinusöl, PEG 20 oder 60 Maisglyceride, PEG 20 oder 60 Mandelglyceride oder PEG 40 Palmkernöl.

5. Zusammensetzung nach einem der Ansprüche 1 bis 4, wobei die Menge des lipophilen, nichtionischen Cotensids in dem isotropen Lösungsmittelgemisch zwischen 10 und 50 Gew.-%, insbesondere zwischen 20 und 40 Gew.-%, wie etwa zwischen 25 und 30 Gew.-% des Gemischs liegt.

6. Zusammensetzung nach Anspruch 1, wobei die Monoglyeridester von C₆₋₂₂-Fettsäuren Glyceryl-Monocaprylat, Glyceryl-Monocaprat, Glyceryl-Monostearat, Glyceryl-Monobehenat sind, oder wobei die Diglyceridester von C₆₋₂₂-Fettsäuren Glyceryl-Dilaurat sind, oder wobei die Mono- und Diglyceridester von C₆₋₂₂-Fettsäuren Capryl-/Caprin-Mono- und Diglyceride oder Glyceryl-Mono- und Diricinoleat sind, oder wobei die Propylenglykolester von C₆₋₂₂-Fettsäuren Propylenglykol-Monocaprylat oder Propylenglykol-Monolaurat sind, oder wobei die Dialkylenglykol-Monoalkylether Diethylenglykol-Monoethylether sind, oder wobei die Polyethylenglykolester eines Triglycerids/Pflanzenöls, 4-8 Mol Ethylenoxidgruppen/Mol Glycerid enthaltend, PEG-6-Maisöl, PEG-6-Mandelöl, PEG-6-Aprikosenkernöl, PEG-6-Olivenöl, PEG-6-Erdnussöl, PEG-6-Palmkernöl oder hydriertes Palmkernöl, PEG-6-Triolein oder PEG-8-Maisöl sind.

7. Zusammensetzung nach einem der Ansprüche 1 bis 6, wobei die Menge C₆₋₂₂-Acylglyerid in dem isotropen Lösungsmittelgemisch zwischen 10 und 70 Gew.-% liegt, z.B. zwischen 15 und 40 Gew.-%, wie etwa zwischen 20 und 30 Gew.-% des Gemischs.

8. Zusammensetzung nach einem der Ansprüche 1 bis 7, wobei das Verhältnis zwischen hydrophilem Tensid:lipophilem Cotensid:C₆₋₂₂-Acylglycerid 2:1:1 beträgt.

9. Zusammensetzung nach einem der Ansprüche 1 bis 8, wobei das isotrope Lösungsmittelgemisch aus Anspruch 1 5 bis 15 Gew.-% oder 8 bis 12 Gew.-%, wie z.B. 10 Gew.-% der Zusammensetzung ausmacht.

10. Zusammensetzung nach einem der Ansprüche 1 bis 9, wobei das C₆₋₂₂-Acylglycerid mittelkettige Triglyceride ist, wobei das Tensid Polyoxyl 40 hydriertes Rizinusöl ist, und wobei das Cotensid Capryl-/Caprin-Mono- und Diglyceride ist, oder wobei das C₆₋₂₂-Acylglycerid langkettige Triglyceride ist, wobei das Tensid Polyoxyl 40 hydriertes Rizinusöl ist, und wobei das Cotensid Capryl-/Caprin-Mono- und Diglyceride ist.

11. Zusammensetzung nach einem der Ansprüche 1 bis 10, wobei der Lipidträger wenigstens ein Paraffin umfasst, das ausgewählt ist aus Paraffinen bestehend aus Kohlenwasserstoffen mit Kettenlängen zwischen C₅ und C₆₀, wobei die Kettenlängen Spitzen aufweisen bei C₁₄₋₁₆, C₁₈₋₂₂, C₂₀₋₂₂, C₂₀₋₂₆, C₂₈₋₄₀ und C₄₀₋₄₄ (gemäß Bestimmung durch Gaschromatographie) oder Mischungen davon.

12. Zusammensetzung nach einem der Ansprüche 1 bis 11, ferner umfassend einen die Viskosität erhöhenden Bestandteil.

13. Zusammensetzung nach Anspruch 12, wobei der die Viskosität erhöhende Bestandteil ein Wachs ist.

14. Zusammensetzung nach einem der Ansprüche 1 bis 13, ferner umfassend ein Silikonwachs oder ein flüchtiges Silikonöl.

15. Zusammensetzung nach Anspruch 14, wobei das flüchtige Silikonöl Cyclomethicon oder Dimethicon ist.

16. Zusammensetzung nach einem der Ansprüche 1 bis 15, ferner umfassend eine nicht reizende Verbindung.

17. Zusammensetzung nach Anspruch 16, wobei die nicht reizende Verbindung Glycerol, Butylenglykol, Sorbitol, Saccharose, Saccharin, Menthol oder Nicotinamid ist.

18. Zusammensetzung nach Anspruch 17, 5 bis 10 Gew.-% Glycerol umfassend.

19. Zusammensetzung nach einem der Ansprüche 1 bis 18, ferner umfassend eine Verbindung, die saure Unreinheiten neutralisieren kann, die für die chemische Stabilität des Vitamin-D-Derivats oder -Analogs in der Zusammensetzung schädlich sind.

20. Zusammensetzung nach Anspruch 19, wobei die Verbindung ein Amin ist, wie etwa Triethanolamin, Trometamol, Monoethanolamin oder Diethanolamin.

21. Zusammensetzung nach einem der Ansprüche 1 bis 20, umfassend 0,001 bis 0,5 mg/g, vorzugsweise 0,002 bis 0,25 mg/g, insbesondere 0,005 bis 0,05 mg/g des Vitamin-D-Derivats oder -Analogs.

22. Zusammensetzung nach einem der Ansprüche 1 bis 21, umfassend:
0,003 - 0,008 % w/w Calcipotriol-Monohydrat;
2 - 3 % w/w mittel- oder langkettige Triglyceride;
2 - 3 % w/w Capryl-/Caprin-Mono- und Diglyceride;
4 - 6 % w/w PEG hydriertes Rizinusöl;
0,5 - 1,5 % w/w Triethanolamin;
85 - 95 % w/w Paraffinträger.

23. Zusammensetzung nach einem der Ansprüche 1 bis 22, ferner umfassend ein oder mehrere zusätzliche therapeutisch wirksame Bestandteile.

24. Zusammensetzung nach Anspruch 23, wobei die zusätzlichen therapeutisch wirksamen Bestandteile ausgewählt sind aus der Gruppe bestehend aus entzündungshemmenden Arzneimitteln wie etwa Kortikosteroiden, wie etwa Betamethason und Ester davon, wie z.B. Valerat oder Dipropionatester, Clobetasol oder Ester davon, wie etwa Propionat, Hydrocortison oder Ester davon, wie etwa Acetat; nichtsteroidale entzündungshemmende Arzneimittel wie etwa Naproxen, Indomethacin, Diclofenac, Ibuprofen, Dexibuprofen, Ketoprofen, Flurbiprofen, Piroxicam, Tenoxicam, lornoxicam oder Nabumeton, Phosphodiesterase-4-Hemmer oder p38-MAP-Kinasehemmer.

25. Zusammensetzung nach Anspruch 24, wobei der zusätzliche wirksame Bestandteil Betamethason oder Ester davon ist, wie z.B. Valerat oder Dipropionatester.

26. Zusammensetzung nach Anspruch 24, wobei der zusätzliche wirksame Bestandteil ein Phosphodiesterase-4-Hemmer ist.

27. Zusammensetzung nach einem der Ansprüche 1 bis 26 zur Verwendung in der Behandlung einer Hauterkrankung oder eines Hautleidens.

28. Zusammensetzung nach Anspruch 27 zur Verwendung in der Behandlung einer Hauterkrankung oder eines Hautleidens, die bzw. das ausgewählt ist aus der Gruppe bestehend aus Psoriasis, Sebopsoriasis, Pustulosis palmoplantaris, Dermatitis, Ichtyosis, Rosacea oder Akne.

## Revendications

1. Composition pharmaceutique sensiblement anhydre pour application cutanée, comprenant de 1 à 20 % en poids d'un mélange de solvants isotropes
(a) d'un tensioactif non ionique hydrophile qui est un ester de polyéthylène glycol d'une huile végétale contenant au moins 20 moles de groupes oxyde d'éthylène/moles de glycéride ;
(b) un co-tensioactif non ionique lipophile qui est choisi dans le groupe constitué d'esters monoglycériques d'acides gras en C₆₋₂₂, d'esters diglycériques d'acides gras en C₆₋₂₂, d'esters mono- et diglycériques d'acides gras en C₆₋₂₂, d'esters de polyéthylène glycol d'acides gras en C₆₋₂₂, d'éthers monoalkyliques de glycol dialkylénique, ou d'esters de polyéthylène glycol d'un triglycéride/huile végétale contenant de 4 à 8 moles de groupes oxyde d'éthylène/moles de glycéride ;
(c) d'un acylglycéride en C₆₋₂₂ qui est un acylglycéride en C₆₋₂₂ non-amphiphile choisi dans le groupe constitué par les huiles végétales hautement purifiées telles que les triglycérides à chaîne moyenne, les triglycérides à chaîne longue ou l'huile de ricin ;
ledit mélange de solvants isotropes pouvant former une microémulsion en présence d'un excès d'eau ;
la composition comprenant en outre un dérivé de la vitamine D ou analogue dissous ou solubilisé dans ledit mélange de solvants isotropes, le dérivé de vitamine D ou analogue étant choisi dans le groupe constitué de calcipotriol, calcitriol, tacalcitol, maxacalcitol, paricalcitol et alfacalcidol, et
un transporteur lipidique sensiblement anhydre pharmaceutiquement acceptable qui est une paraffine choisie parmi les hydrocarbures ayant des longueurs de chaîne comprises entre C₅ et C₆₀.

2. Composition selon la revendication 1, l'analogue de la vitamine D étant du calcipotriol ou du monohydrate de calcipotriol.

3. Composition selon la revendication 1 ou 2, la quantité de tensioactif hydrophile dans le mélange de solvants isotropes étant comprise entre 10 et 70 % en poids, en particulier entre 30 et 60 % en poids, notamment entre 40 et 50 % en poids du mélange.

4. Composition selon la revendication 3, l'ester de polyéthylène glycol d'une huile végétale contenant au moins 20 moles de groupes oxyde d'éthylène/moles de glycéride étant choisi dans le groupe constitué de dérivés de l'huile de ricin de polyoxyéthylène, p. ex. huile de ricin PEG 20, 30, 35, 38, 40, 50 ou 60 ou huile de ricin hydrogénée PEG 20, 25, 30, 40, 45, 50, 60 ou 80, glycérides de maïs PEG 20 ou 60, glycérides d'amande PEG 20 ou 60 ou huile de palmiste PEG 40.

5. Composition selon l'une quelconque des revendications 1 à 4, la quantité de co-tensioactif non ionique lipophile dans le mélange de solvants isotropes étant comprise entre 10 et 50% en poids, en particulier entre 20 et 40% en poids, notamment entre 25 et 30% en poids du mélange.

6. Composition selon la revendication 1, les esters monoglycériques d'acides gras en C₆₋₂₂ étant du monocaprylate de glycéryle, monocaprate de glycéryle, monostéarate de glycérol, monobéhénate de glycéryle, ou les esters diglycériques d'acides gras en C₆₋₂₂ étant du dilaurate de glycéryle, ou les esters mono- et diglycériques d'acides gras en C₆₋₂₂ étant des mono- et diglycérides capryliques/capriques ou des mono- et diricinoléate de glycérol, ou les esters de polyéthylène glycol d'acides gras en C₆₋₂₂ étant du monocaprylate de propylèneglycol ou du monolaurate de propylèneglycol, ou les éthers monoalkylés de dialkylène glycol étant des éthers mono-éthyliques de diéthylène glycol, ou les éthers de propylèneglycol d'un triglycéride/huile de légume contenant de 4 à 8 moles de groupes oxyde d'éthylène/moles de glycéride étant de l'huile de maïs PEG-6, de l'huile d'amande PEG-6, de l'huile de noyau d'abricot PEG-6, de l'huile d'olive PEG-6, de l'huile d'arachide PEG-6, de l'huile de palmiste PEG-6 ou de l'huile de palmiste hydrogénée, de la trioléine PEG-6 ou de l'huile de maïs PEG-8.

7. Composition selon l'une quelconque des revendications 1 à 6, la quantité d'acylglycéride C₆₋₂₂ dans le mélange de solvants isotrope étant comprise entre 10 et 70 % en poids, par exemple entre 15 et 40 % en poids, notamment entre 20 et 30 % en poids du mélange.

8. Composition selon l'une quelconque des revendications 1 à 7, le ratio tensioactif hydrophile:co-tensioactif lipophile:acylglycéride en C₆₋₂₂ étant 2:1:1.

9. Composition selon l'une quelconque des revendications 1 à 8, le mélange de solvants isotropes selon la revendication 1 constituant de 5 à 15 % en poids, de 8 à 12 % en poids ou de 9 à 11 % en poids, par exemple 10 % en poids de la composition.

10. Composition selon l'une quelconque des revendications 1 à 9, l'acylglycéride en C₆₋₂₂ étant des triglycérides à chaîne moyenne, le tensioactif étant de l'huile de ricin hydrogénée polyoxyl 40 et le co-tensioactif étant des mono- et diglycériques capryliques/capriques, ou l'acylglycéride en C₆₋₂₂ étant des triglycérides à chaîne longue, le tensioactif étant de l'huile de ricin hydrogénée polyoxyl 40, et le co-tensioactif étant des mono- et diglycériques capryliques/capriques.

11. Composition selon l'une quelconque des revendications 1 à 10, le transporteur lipidique comprenant au moins une paraffine choisie parmi des paraffines comprenant des hydrocarbures à longueurs de chaîne comprises entre C₅ et C₆₀, les longueurs de chaîne ayant un pic en C₁₄₋₁₆, C₁₈₋₂₂, C₂₀₋₂₂, C₂₀₋₂₆, C₂₈₋₄₀ et C₄₀₋₄₄ (tel que déterminé par chromatographie en phase gazeuse) ou leurs mélanges.

12. Composition selon l'une quelconque des revendications 1 à 11, comprenant en outre un ingrédient augmentant la viscosité.

13. Composition selon la revendication 12, l'ingrédient augmentant la viscosité étant une cire.

14. Composition selon l'une quelconque des revendications 1 à 13, comprenant en outre une cire silicone ou une huile volatile silicone.

15. Composition selon la revendication 14, l'huile volatile silicone étant de la cyclométhicone ou de la diméthicone.

16. Composition selon l'une quelconque des revendications 1 à 15, comprenant en outre un composé anti-irritation.

17. Composition selon la revendication 16, le composé anti-irritation étant du glycérol, du butylène glycol, du sorbitol, du saccharose, de la saccharine, du menthol ou de la nicotinamide.

18. Composition selon la revendication 17, comprenant de 5 à 10 % en poids de glycérol.

19. Composition selon l'une quelconque des revendications 1 à 18, comprenant en outre un composé capable de neutraliser les impuretés acides préjudiciables à la stabilité chimique du dérivé de la vitamine D ou analogue dans la composition.

20. Composition selon la revendication 19, ledit composé étant une amine telle que triéthanolamine, trométamol, monoéthanolamine ou diéthanolamine.

21. Composition selon l'une quelconque des revendications 1 à 20, comprenant de 0,001 à 0,5 mg/g, de préférence de 0,002 à 0,25 mg/g, en particulier de 0,005 à 0,05 mg/g, de dérivé de la vitamine D ou analogue.

22. Composition selon l'une quelconque des revendications 1 à 21, comprenant :
de 0,003 à 0,008 % en poids de monohydrate de calcipotriol
de 2 à 3 % en poids de triglycérides à chaîne moyenne ou longue
de 2 à 3 % en poids de mono- et diglycérides capryliques/capriques
de 4 à 6 % en poids d'huile de ricin hydrogénée PEG 40
de 0,5 à 1,5 % en poids de triéthanolamine
de 85 à 95 % en poids de porteur de paraffine

23. Composition selon l'une quelconque des revendications 1 à 22, comprenant en outre un ou plusieurs ingrédients supplémentaires thérapeutiquement actifs.

24. Composition selon la revendication 23, ces ingrédients actifs supplémentaires étant choisis dans le groupe constitué des médicaments anti-inflammatoires tels que les corticostéroïdes, comme la bétaméthasone et ses esters, par exemple l'ester de valérate ou dipropionate, le clobétasol ou ses esters, comme le propionate, l'hydrocortisone ou ses esters, tels que l'acétate ; de médicaments anti-inflammatoires non stéroïdiens tels que le naproxène, l'indométacine, le diclofénac, l'ibuprofène, le dexibuprofène, le kétoprofène, le flurbiprofène, le piroxicam, le ténoxicam, le lornoxicam ou le nabumeton, les inhibiteurs de la phosphodiestérase 4 ou les inhibiteurs de la kinase p38 MAP.

25. Composition selon la revendication 24, l'ingrédient actif supplémentaire étant la bétaméthasone ou ses esters, par exemple les esters de valérate ou dipropionate.

26. Composition selon la revendication 24, l'ingrédient actif supplémentaire étant un inhibiteur de la phosphodiestérase 4.

27. Composition selon l'une quelconque des revendications 1 à 26 destinée à être utilisée dans le traitement d'une maladie ou d'un trouble cutané.

28. Composition selon la revendication 27, à utiliser dans le traitement d'une maladie ou d'un trouble cutané choisi dans le groupe constitué par le psoriasis, le sébopsoriasis, la pustulose palmoplantaire, la dermatite, l'ichtyose, la rosacée ou l'acné.
